# EUROPEAN PATENT APPLICATION

(11) **EP 2 689 775 A1**
(43) Date of publication of application: **29.01.2014**
(21) Application number: 12177817.9
(22) Date of filing: 25.07.2012
(51) Int. Cl.: A61K 9/00, A61K 39/095, A61K 39/39

(54) **Cochleate with only one mamp**

(71) Applicant: Instituto Finlay, Centro de Investigacion-Produccion de vacunas y sueros, La Coronela La Lisa Ciudad Habana (CU); CENTRO DE BIOACTIVOS QUIMICOS, Santa Clara 50100 (CU)
(72) Inventor: Perez Martin, Oliver Germán, 11300 La Habana (CU); Gonzalez Aznar, Elizabeth, 11300 La Habana (CU); Cabrera Blanco, Osmir, 11600 La Habana (CU); Zayas Vignier, Caridad, 11600 La Habana (CU); Sifontes Rodriguez, Sergio, 50100 Villa Clara (CU); Balboa Gonzalez, Julio Antonio, 1360 La Habana (CU); Cuello Perez, Maribel, 11600 La Habana (CU); Romeu Alvarez, Belkis, 10700 La Habana (CU); Lastre Gonzalez, Miriam de San Juan Bosco, 11300 La Habana (CU); Rodriguez Negrin, Zenaida, 52800 Villa Clara (CU); Solis Rodriguez, Rosa Lydia, 11600 La Habana (CU)
(74) Representative: Jansen, Cornelis Marinus

(57) **Abstract**

The present invention relates to the field of immunology, specifically the branch of adjuvants and vaccines. The technical objective is to obtain adjuvants based on cochlear structures that are neither proteolipidic nor lipidic and that contain microbe-associated molecular patterns (MAMPs) and have the optional addition of antibiotics/chemotherapeutic agents with the aim of using them as vaccine adjuvants for the treatment and prevention of veterinary and human diseases. Additionally, these would be used as immunopotentiators in veterinary and human formulations. It is also a technical objective of this patent to utilize the said new cochlear structures with particular antigens added or liberated in vivo for the combined effect of immunopotentiation and antibiotics/chemotherapeutic agents in infectious diseases to obtain therapeutic vaccines for veterinary and human use in the pharmaceutical industry. The invention also relates to the method for obtaining sterile cochlear structures.

## Description

### Technological Field

This invention relates to the field of immunology, specifically the branch of adjuvants and vaccines. The technical objective is to obtain adjuvants based on cochlear structures that are neither proteolipidic nor lipidic and that contain microbe-associated molecular patterns (MAMPs) and have the optional addition of antibiotics with the aim of using them as vaccine adjuvants for the treatment and prevention of veterinary and human diseases. Additionally, these will be used as immunopotentiators in veterinary and human formulations. It is also a technical objective of this patent to utilize said new cochlear structures with particular antigens added or liberated in vivo for the combined effect of immunopotentiation and antibiotics/chemotherapeutic agents in infectious diseases to obtain therapeutic vaccines for veterinary and human use in the pharmaceutical industry. The invention also relates to the method for obtaining sterile cochlear structures.

### State of the Art

Adjuvants are substances that promote a specific immune response against an antigen, provoking a more rapid induction of this response and increasing its duration (Vogel FR. Dev. Biol. Stand. 1998,92:241-248). Alumina continues to be the adjuvant that is most used in parenteral vaccines. Nevertheless, this is not a powerful adjuvant that could help new subunit vaccines. It is considered to only have delivery-system properties and, recently, a new mechanism of action independent of the Toll-like receptors that involves Nalp3 (Eisenbarth SC et al. Nature, 2008,453:1122-1126) has been described. Its mucosal usefulness has also not been proven and it is assumed that it preferentially induces a Th2 response. There is a scarcity of licensed vaccine adjuvants (MF59, AS02, Virosoma, AFPL1, Proteollin, MPL, etc.), few are for mucosal application, and of these, none are licensed for use in humans (mutants of LT (LT63K, LTR72), CpG, Chitosan, ISCOM and AFCo1) (Singh M and O'Hagan DT. Pharm. Res. 2002, 19(6):715-728, Pérez O et al. Immunology and Cell Biology. 2004,82:603-610). The adjuvants are involved in: stimulating the innate response (immunopotentiators); adequately distributing the antigens (delivery system) (Pashine A Valianti NM. Nat. Med. 2005, 11:S63-68; Singh M and O'Hagan DT. Pharm. Res. 2002,19(6):715-728), and directing the immune response toward desired protective responses (Immunopolarization) (O Pérez, et al. PharmacologyOnline, 2006,3:762-64). The development of immunopotentiators or delivery systems that are effective for mucosal use is another main objective of development of vaccines and of pharmaceutical companies worldwide. These have centered on the search for adjuvant systems that combine one or various immunopotentiators with an adequate delivery system. We, on the other hand, have based our work on proteoliposomes and proteoliposome-derived cochleates which contain various immunopotentiators that act in a synergistic manner, have a capacity for delivery, and polarize the immune response preferentially toward Th1 patterns and also induce cytotoxic responses (CTL, Pérez O et al. Scand J Immunology 2007,66:271-77; certified by the author of the invention OCPI 23313, 2008 Pérez Martin OG et al.; WO/2004/047805; WO/2003/094964 and EP1716866). The cochleates that we developed previously are derived from proteoliposomes (vesicles of external membranes, fundamentally of bacteria) which contain proteins and lipids (proteolipidic structures) and were directed toward obtaining powerful mucosal adjuvants. For the latter point, sterility was not an essential requirement. Around the world, as well, cochleates have been obtained from synthetic or purified lipids (Gould-Fogerite et al. U. S. Pat. No 5, 643, 574, July 1, 1997; Zarif, Methods Enzymol 2005,391:314-29; Miclea, Varma et al. Biochim Biophys Acta 2007, 1768(11):2890-8; and Syed, Woo et al. Int J Pharm 2008, 363(1-2): 118-25).

### DETAILED DESCRIPTION OF THE INVENTION

The present invention includes new cochlear structures that do not originate from proteolipidic nor lipidic structures and that have adjuvant capabilities to contain single or multiple MAMPs with synergistic action. Toxic MAMPs are previously detoxified and, optionally, there is also the inclusion of potentially toxic antibiotics whose toxicity is reduced inside these cochlear structures. The addition of particular antigens is also optional, and these antigens can come from the action of antibiotics on pathogens that infect the patient specifically, and once these antigens are immunopotentiated through MAMPs they are converted into therapeutic vaccines or immunotherapeutic products. The objective of the present invention is to obtain new cochlear structures that are not proteolipidic nor lipidic and that contain MAMPs with the optional addition of antibiotics/chemotherapeutic agents with the aim of using them as vaccine adjuvants for the treatment and prevention of veterinary and human diseases. Additionally, these will be used as immunopotentiators in veterinary and human formulations. It is also an objective of this patent to utilize said novel cochlear structures with particular antigens added or liberated in vivo for the combined effect of immunopotentiation and antibiotics in infectious diseases to obtain therapeutic vaccines for veterinary and human use in the pharmaceutical industry. The invention also relates to the method for obtaining sterile cochlear structures.
The Adjuvant Finlay Cochleate, hereafter referred to as AFCo3, unlike the previous Finlay adjuvants, does not originate from proteoliposomes (proteolipidic structures) containing only LPS as a MAMP.
"Proteoliposomes" refers to particles obtained from the external membrane of bacteria using various methods such as: their isolation without detergent; a process that includes detergent (such as deoxycholate, SDS, etc.), or extraction based on vesicles ("bleb") of supernatants of cultures, and, in particular, those revealed in US 5,597,572. The proteoliposomes are made up of abundant proteins inserted in the lipidic structure. In contrast to the previous ones, "lipidic" structures here refer to cochleates and liposomes formed from negative lipids.
"MAMP" (microbe-associated molecular pattern) refers to molecular structures maintained in microbes, whether pathogenic or not, that are capable of stimulating the innate immune system and inducing a powerful adaptive response while also being involved in polarizing the immune response.
"Non-proteolipidic, non-lipidic cochleates" refers to cochleates that are not derived from a proteoliposome as is referred to in the patent request (WO/2004/047805)), Pérez O et al. Infect Immun. 2001, 69(7):4502-4508 and Pérez O et al. Immunology and Cell Biology. 2004, 82:603-610) nor to the products made from synthetic lipids (Gould-Fogerite et al. U. S. Pat. No 5, 643, 574, July 1, 1997). In addition, for the effects of this patent, the concept of cochleates that are neither proteolipidic nor lipidic extends to the production of the same based on the use of purified MAMPs, obtained in recombinant or synthetic form, wherein the toxins are detoxified and either just one or various ones, but with synergistic action, are included. The inclusion of another PAMP, flagellin, was efficiently incorporated as the LPS-flagellin combination. "Purified MAMPs" refers to the use of MAMPs which have the characteristic of not being toxic and can, therefore, be directly included into AFCo3. This concept extends to the combination of various purified MAMPs, and to the combination of those that are purified, recombinant, and detoxified.
"Detoxified MAMPs" refers to the use of MAMPs which, because of their toxic characteristics such as lipopolysaccharide (LPS or lipooligosaccharide, LOS) derived from Neisseria meningitidis serogroup B, detoxification is required for their inclusion into AFCo3 and for achieving decreased toxicity.
"Antibiotics/chemotherapeutic agents" refers to synthetic or conventional products including, but not limited to, 2-bromo-5-(2-bromo-2-nitrovinyl)-furan (G1) and Amphotericin B, whose toxicity is reduced by the incorporation into AFCo3 and this, at the same time, serves as their vehicle.
"Treatment of diseases" refers to chronic diseases including, but not limited to, tuberculosis, Acquired Immune Deficiency Syndrome (A.I.D.S.), leishmaniasis, and malaria.
"Particular antigens" refers to protective antigens of bacteria, viruses, protozoans, fungi, and helminths obtained through any method for their use in vaccines.
The use of adjuvants containing various MAMPs with synergistic action is desirable when powerful adjuvants are required. The line of adjuvant systems of GlaxoSmithKline is based on the combination of various compounds with an adjuvant effect, while the "Finlay adjuvants" derived from proteoliposomes are a good example of synergistic action between various MAMPs. Nevertheless, contrary to those previously described by Pérez O et al., the present invention finds that AFCo3, whose only MAMP is purified LPS of N. meningitidis serogroup B, is able to develop a similar response to that induced by AFCo1 which contains various MAMPs with synergistic action. It was also found that detoxified LPS of N. meningitidis B could induce powerful responses. It was found that there was also no need for other lipids and that only one conformational change induced by the interactions of sodium deoxycholate (DOC) and calcium (Ca2+) with a single MAMP, LPS, was as powerful. It should be noted that LPS contains the lipid A but this is not required in order to obtain the cochlear structures for which DOC and Ca2+ are sufficient.
The mucosal and parenteral systems are organized in different ways and the mucosal system is more compartmentalized. Licensed mucosal adjuvants do not even exist in human vaccines and it is more difficult to induce a response mucosally than parenterally (Bouvet, J P et al. I & I. 1994, 62:3957-3961 and Nardelli-Haefliger, DR, et al. J. Virol. 1999, 73:9609-9613). It was found that AFCo3 applied nasally was as efficient as AFCo1 applied nasally or parenterally.
It was also found that the combined intranasal and subcutaneous treatment with AFCo3 containing G1 was effective for the treatment of homing pigeons (Columba livia) that were severely infected in a natural manner with the avian pox virus.
The present invention also includes the obtaining of different vaccine formulations that make use of the capacity of AFCo3 to function both ways, i.e. mucosally and parenterally, in order to induce Th1 responses with CTL activity and to be applied in a strategy for one-time vaccines ("Single Time Vaccination Strategy").
The immunopotentiatory property of the adjuvants for stimulating the innate response, with their mucosal applicability, particularly the oral applicability, are put to use in the present patent, and for what also forms part of the present invention, the obtainment of immunopotentiatory formulations applied in animal food, particularly fish, chicken, pigs, etc. via the branchial, oral, or parenteral routes. The adjuvation of the immune response in fish was evaluated by the induction of specific IgM in fish immunized with an oral dosage and simultaneous parenteral administration.
Antigens with a low degree of immunogenicity such as ovalbumin (OVA) and bovine serum albumin (BSA) or with a higher degree of immunogenicity such as tetanus toxoid were efficiently adjuvated by AFCo3 when said applications were applied parenterally and mucosally in mice.
The vaccine formulations adjuvated with AFCo3 of the present invention can be used in prophylactic vaccines for use in humans and animals, as well as in therapeutic vaccines against chronic human and veterinary infections, particularly when combining the lytic action of antibiotics/chemotherapeutic agents on infections, particularly chronic infections, and the immunopotentiatory and immunopolarizing capabilities of MAMPs included in cochlear structures, particularly, but not limited to, AFCo3.
The novelty of the present invention lies in, primarily, the obtaining of a cochlear structure which is not derived from proteolipidic nor lipidic structures, with adjuvant activity when applied mucosally and/or parenterally for immunization.
It is particularly novel that with only one MAMP, i.e. LPS, responses are obtained which are similar to those obtained with AFCo1 which contains various MAMPs with synergistic action at the mucosal and parenteral level.
It is novel to use the immunopotentiatory capability of AFCo3 to stimulate the innate and acquired response, particularly important in fish.
It is novel to have managed to include G1 in AFCo3, reducing its toxicity.
The method of production is also novel, because it does not include proteolipidic nor lipidic structures, and this enables the reduction of its own costs and increased sterile production, and expands the possibilities for mucosal application of our previous cochleates (AFCo1 and AFCo2) to parenteral application and the combination of routes for administration of single-time vaccines.
The proposed solution has the following advantages:
Various adjuvants can be produced by the inclusion of one or various MAMPs with synergistic action in AFCo3;
It can be used in prophylactic and therapeutic vaccine formulations and since the cost for obtaining the cochleates is reduced, this allows for less-costly obtainment of adjuvants and vaccines;
It can be used as an oral immunopotentiator or as an aerosol immunopotentiator in veterinary diseases;
It can be used as an immunopotentiator in infectious diseases in humans, particularly in chronic diseases, applied via various administration routes; Antibiotics can be included to reduce its toxicity or improve its delivery; Through AFCo3, specific responses of IgAS, subclasses of IgG Th1 are induced when applied via the mucosal route, parenteral route, or simultaneously via both routes;
Immunotherapeutic products are able to be produced in the absence of external antigens, making use of the lytic capability of the antibiotics on infectious microorganisms and immunopotentiatory properties of AFCo3 that permit the liberation of antigens of infected cells and the appropriate, specific immune response;
The process of obtaining the adjuvant is simplified, reducing its production cost without affecting the potency and Th1 polarization of the response;
A sterile production process is achieved, thereby amplifying the use of obtained adjuvants, as well as vaccine compositions with their parenteral use and the possibility of combining mucosal and parenteral routes in order to obtain immune responses that are qualitatively superior with decreased dosages.

### Brief description of the figures

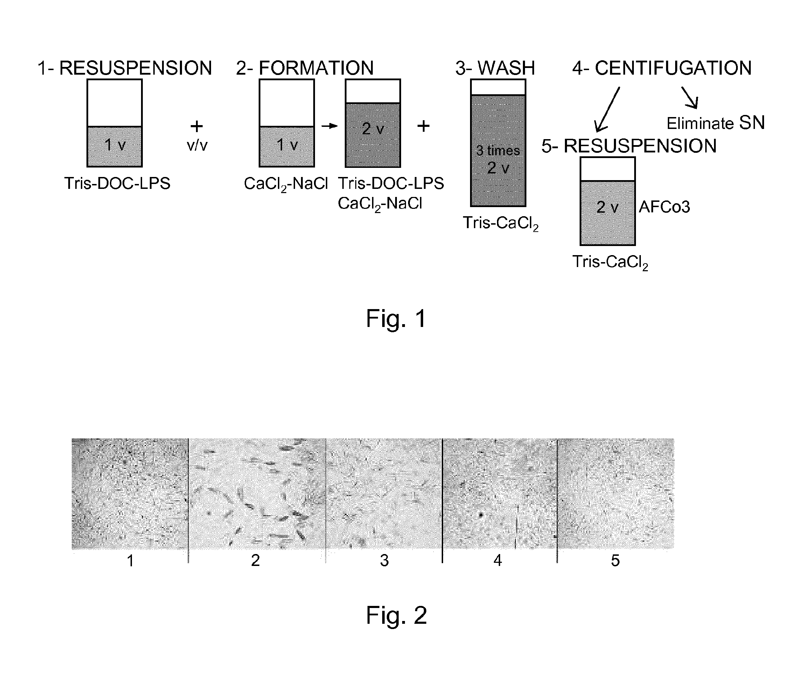

Figure 1. Obtaining AFCo3LPS. In a closed-loop process and under sterile conditions, AFCo3 was obtained in a total of 5 continuous steps. 1-Resuspension. The concentrations of LPSd (detoxified lipopolysaccharide) are adjusted to 2 mg/mL and to 40 µg/mL for LPSsd (non-detoxified lipopolysaccharide), in Resuspension Solution. 2-Formation. Under continuous agitation, Formation Solution is added v/v at a drip rate of 2.2 mL /minute. 3-Wash. Next, under the same conditions of continuous agitation, the Wash Solution is added at a drip rate of 5 mL/min at the rate of 3 times to volume of Formation. 4-Centrifugation. After 30 min. of rest without agitation, the entire formulation is centrifuged at 1500 rpm/min, 15 min, at 4°C (4). 5-Final Formulation. The SN (supernatant) is completely discarded and the sediment ("pellet", cochlear structures) is resuspended in Tris-CaCl2 in volume equal to Formation. The formation and presence of cochlear structures were observed in each step by the presence of a white precipitate and verified by observation under an optical microscope.
Figure 2. Optical microscope. Variants of AFCo3LPSd (2h-row 2, 4h-row 3 and 6h-row 4) and AFCo3LPSsd (row 3) were observed under optical microscope (20X lens) in order to confirm the presence of tubular structures between 5 and 10 µm using AFCol (row 1) as a positive control.

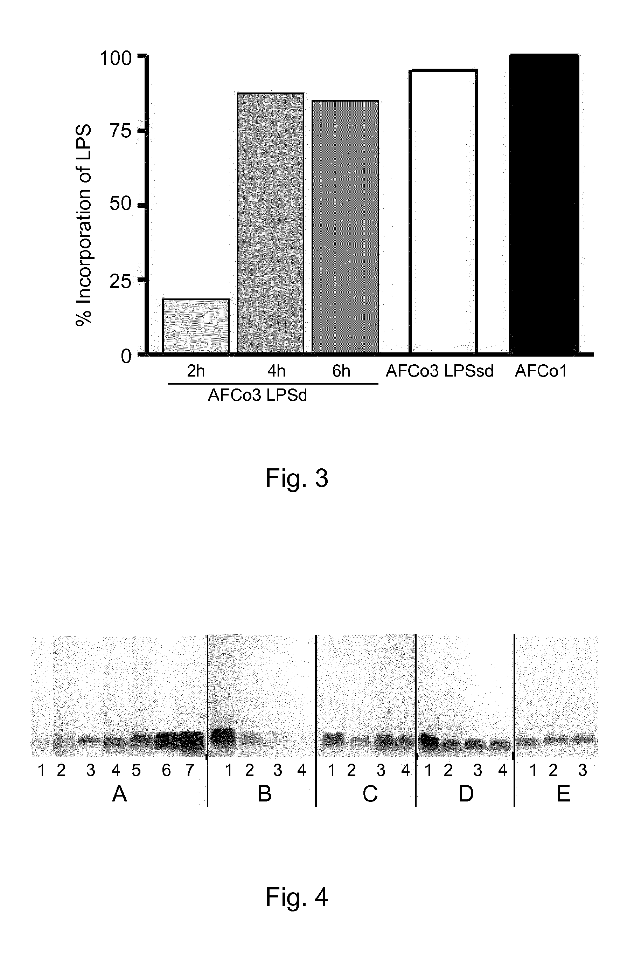

Figure 3. Percentage of incorporation of LPS in AFCo3. The percentage of incorporation of LPS in AFCo3 (B) was figured using the calculation ((I x 100)/T) wherein I is the concentration of LPS in the precipitate and T is the concentration of LPS in total.
Figure 4. Electrophoretic evaluation. Electrophoresis was performed in sodium dodecyl sulfate polyacrylamide gel (SDS-PAGE), using a gel concentrator at 5% (m/v) and gel separator at 15% (m/v). Silver-stain was utilized according to Tsai CM et al. (A) Curve of native lipopolysaccharide (LPS) of N. meningitidis, from 10 µg/mL (1) to 1 mg/mL (7). (B) AFCo3LPSd (detoxified) 2h, (C) AFCo3LPSd 4h, (D) AFCo3LPSd 6h and (E) AFCo3LPSsd. The numbers signify the stages of the process for obtaining AFCo3 during which the sample was analyzed: 1) Resuspension, 2) Formation, 3) Wash and 4) Incorporation.

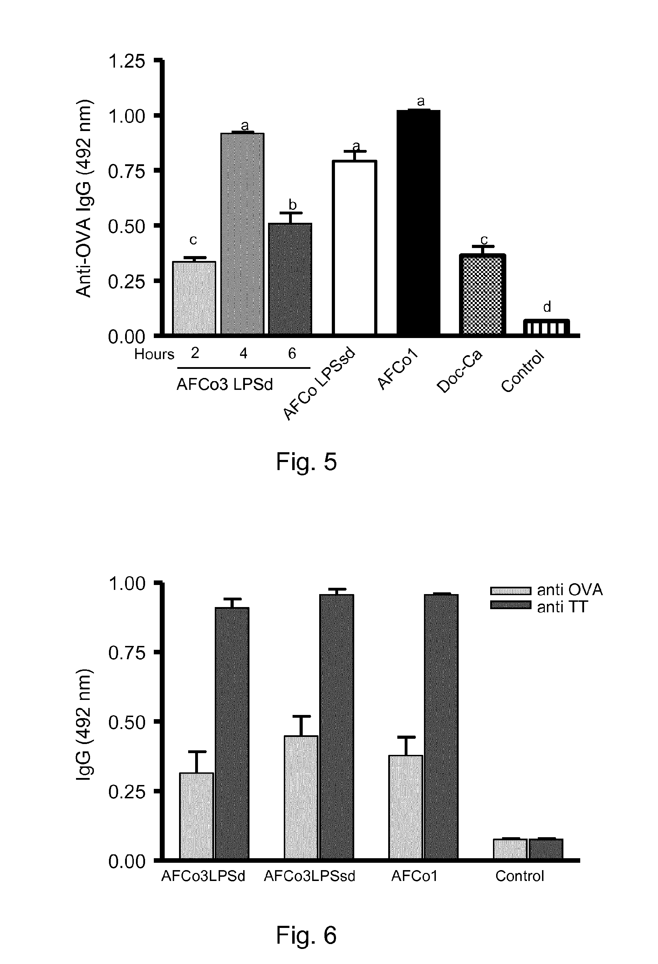

Figure 5. Immunogenicity of the variants of AFCo3 containing LPS of Neisseria meningitidis serogroup B detoxified (LPSd). BALB/c mice were immunized with 2 doses i.m. (50 µL each) of OVA (10 µg) at 0 and 14 days, coadministered with 25 µg of AFCo3LPSd (variants 2, 4, and 6 hrs.) and 0.5 µg of AFCo3LPSsd. AFCo1 (0.5 µg of LPS) was used as a positive control and DOC-Ca was used as a negative control. The evaluation of the levels of anti-OVA IgG was performed using ELISA on serum extracted 21 days after the last dose. The figure shows the averages and standard deviations of the mathematic relationship of the values (optical density) of 2 determinations in 3 independent experiments. The significant differences between the averages of the different groups were determined by a multiple comparison test (Tukey), present in the Graph Pad Prism 4 software (Calif.). The P <0.05 value was considered to be statistically significant and the value was represented by different letters: a (p<0.001); b (p<0.05) and c (p <0.01).
Figure 6. Anti-OVA and anti-TT immune response induced by the intranasal administration of AFCo3LPSd 4 h. C57BL/6 mice were immunized with three intranasal doses (i.n., 12.5 µL, via the nasal cavity) of OVA (20 µg) or tetanus toxoid (TT, 5 Lf (Limit of Flocculation)) at 0, 7, and 14 days, adjuvated with AFCo3LPSd 4h (25 µg); AFCo3LPSsd (2 µg) and AFCo1 (2 µg of LPS). As a control, one group was not immunized. The evaluation of the levels of anti-OVA and anti-TT IgG was performed using ELISA on serum extracted 21 days after the last dose. The figure shows the averages and standard deviations of the mathematic relationship of the values (optical density) of 2 determinations in 3 independent experiments. The significant differences between the averages of the different groups were determined by a multiple comparison test (Tukey), present in the Graph Pad Prism 4 software (Calif.).

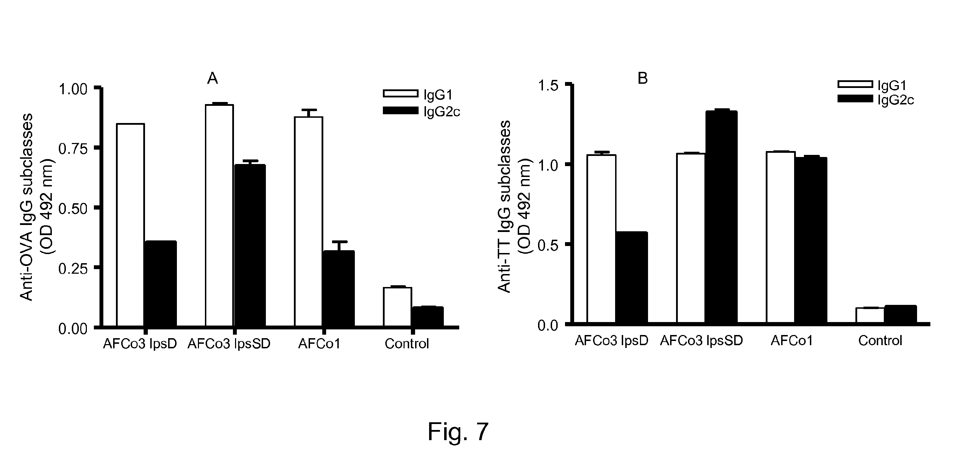

Figure 7. Response of anti-OVA and anti-TT IgG1 and IgG2c subclasses induced by the intranasal administration of AFCo3LPSd 4 h. C57BL/6 mice were immunized with three intranasal doses (i.n., 12.5 µL, via the nasal cavity) of OVA (20 µg) or tetanus toxoid (TT, 5 Lf (Limit of Flocculation)) at 0, 7, and 14 days, adjuvated with AFCo3LPSd 4h (25 µg); AFCo3LPSsd (2 µg) and AFCo1 (2 µg of LPS). As a control, one group was not immunized. The evaluation of the levels of anti-OVA and anti-TT IgG1 and IgG2C was performed using ELISA on serum extracted 21 days after the last dose. The figure shows the averages and standard deviations of the mathematic relationship of the values (optical density) of 2 determinations in 3 independent experiments.

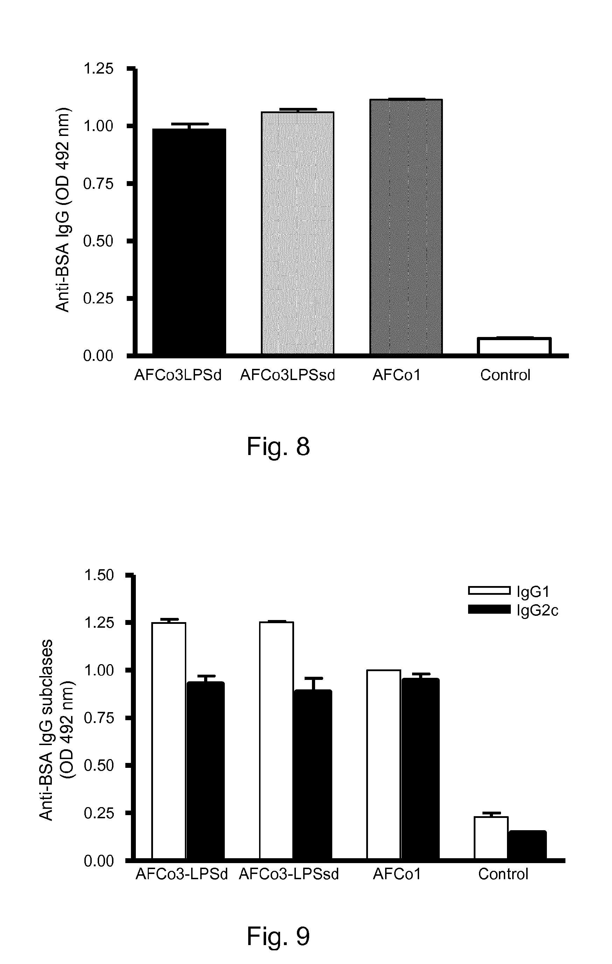

Figure 8. Anti-BSA immune response induced by the intramuscular administration of AFCo3LPSd 4h. C57BL/6 mice were immunized with 2 i.m. doses of BSA (20 µg) at 0 and 14 days, adjuvated with AFCo3LPSd 4h (25 µg); AFCo3LPSsd (0.5 µg) and AFCo1 (0.5 µg of LPS). The control group of animals was not immunized. The evaluation of the levels of anti-BSA IgG was performed using ELISA on serum extracted 21 days after the last dose. The figure shows the averages and standard deviations of the mathematic relationship of the values (optical density) of 2 determinations in 3 independent experiments. The significant differences between the averages of the different groups were determined by a multiple comparison test (Tukey), present in the Graph Pad Prism 4 software (Calif.).
Figure 9. Response of anti-BSA IgG1 and IgG2c subclasses induced by the intramuscular administration of AFCo3-LPSd 4h. C57BL/6 mice were immunized with 2 i.m. doses of BSA (20 µg) at 0 and 14 days, adjuvated with AFCo3LPSd 4h (25 µg); AFCo3LPSsd (0.5 µg) and AFCo1 (0.5 µg of LPS). The control group of animals was not immunized. The evaluation of the levels of anti-BSA IgG1 and IgG2c was performed in serum extracted 21 days after the last dose using ELISA. The figure shows the averages and standard deviations of the mathematic relationship of the values (optical density) of 2 determinations in 3 independent experiments.

### EXAMPLES OF IMPLEMENTATION

The present invention will be described through the following specific examples.

### Example 1. Purification of Lipopolysaccharide (LPS) of Neisseria meningitidis serogroup B

The purification of LPS of N. meningitidis (serogroup B Cu385-83 strain B: 4:P1.19.15) was achieved through extraction with phenol, chromatography, and ultracentrifugation (Balboa JA et al. Vaccimonitor [online]. 2008, 17(1):17-26). In short, the process begins with the supernatant (SN) from the ultracentrifugation of the production process of the anti-meningococchal Cuban vaccine VA-MENGOC-BC®, which is performed under Good Manufacturing Practice ("GMP") at the Finlay Institute. Through this process, the proteins that were purified through chromatography are separated in order to form the proteoliposome (PL) of the vaccine. From this supernatant, precipitation with ethanol 80%, extraction of the proteins with phenol 90% between 65-70°C, and fractionated ultracentrifugation at 105000 x g are achieved. Three lots of LPS are obtained; 1069 g in total, with a content of proteins, nucleic acids, and sialic acid of 0.5%, 0.3%, and 2.2% (m/m), respectively, with respect to LPS. The evaluation with chromatography showed high molecular integrity, with reproducible distribution constant values (0.36-0.38) and a possible association of sialic acid to LPS. There was observed homogeneity in the electrophoretic profiles of the three lots and high endotoxic activity in the mice. The purified LPS was identified as that of immunotype L3,7,9. The purification process that was used allowed for making use of a collateral fraction of the production process of the vaccine, was scalable, did not include chromatography methods, and enabled the obtainment of a large quantity of LPS which was not available on the market and that had a high degree of purity and high endotoxic activity.

### Example 2. Detoxification by n-deacetylation of lipopolysaccharide (LPS) of Neisseria meningitidis serogroup B

For n-deacetylation, 20 mg of LPS were dissolved in 5 mL of sodium hydroxide (NaOH) in concentrations of 0.7 N and placed into an oven at 100°C for 2, 4, or 6 hrs. (to obtain three different levels of detoxification). Afterwards, it was cooled to room temperature and the pH was neutralized with hydrochloric acid 1:2 (v/v). The presence of free amine groups in the LPS was determined through the o-Phthaldialdehyde spectrophotometric assay technique (OPA, Church FC et al. Anal Biochem 146(2):343-348, 1986), and the result showed the generation of 3.9 times the amine groups present in the original LPS (Table 1).

**Table 1. Determination of amine groups generated in the lipopolysaccharide (LPS) of the Neisseria meningitidis serogroup B product of the n-deacetylation of the fatty acids of Lipid A through o-Phthaldialdehyde spectrophotometric assay technique (OPA).**

| **Samples** | **Hydrolysis time (hrs.)** | **Groups NH2 (mMoles/mg)** |
|---|---|---|
| Native LPS | 0 | 0.61 |
| Detoxified LPS | 4 | 2.4 |

### Example 3. Determination of the toxicity of the lipopolysaccharide (LPS) of N. meningitidis serogroup B using the Limulus Amebocyte Lysate (LAL) method

Two lots of native (not detoxified) LPS N. meningitidis and LPS detoxified for 4 hrs. were analyzed. The toxicity of the LPS was determined using the chromogenic method of the Limulus Amebocyte Lysate (LAL) assay, Coatest EndotoxinE kit, of the Swedish company Chromogenix AB. The assay was carried out according to the techniques of the manufacturer, with a master curve between 0.15 and 1.2 EU (Endotoxin Units) in test tubes using a Stuart Scientific dry block heater for 24 tubes. In order to carry out the method, borosilicate 10 x 75 mm reaction tubes (Pyrotubes&commat) of the Associates of Cape Cod company, 15 mL Costar brand polystyrene centrifuge tubes for the master curve, and Rainin pipette tips were used. All of these materials were free from endotoxins.
The results of the assay showed that the native LPS had values of 12140 and 4220 EU/µg while the detoxified LPS reduced its values to 5.0 and 6.5, respectively.
Conclusion: The n-deacetylation process succeeded in detoxifying the LPS.

### Example 4. Obtaining the AFCo3LPSd

The process began with three variants (2, 4, and 6 hours) of detoxified LPS (LPSd) and LPS that was not detoxified (LPSsd), and these three variants were evaluated to check their LPS concentration through the determination of 2-keto-3-deoxyoctonoic acid (KDO) using the thiobarbituric acid (TBA) method (Osborn MJ. Proc. Natl. Acad. Sci. USA. 1963;50:499-506). The concentrations were adjusted to 2 mg/mL for the LPSd variants and to 40 µg/mL for the LPSsd, in a resuspension solution (Tris 30 mM, 1% sodium deoxycholate (DOC)); this process was named "Resuspension". The formulations that were thereby obtained were filtered through a filter with pore size of 0.2 µm. Through a process of continuous agitation (330 rpm) and slow dripping (2.2 mL/min), the Formation solution (CaCl2 10mM, NaCl 100mM) was added. The formation of cochlear structures was evidenced by the formation of a white precipitate and subsequent observation with an optic microscope. This process was named "Formation". Once the cochlear structures are formed, the process continues to the "Wash" step. In order to achieve this step, the Wash solution (Tris 10 mM, CaCl2 5mM) was added under a process of continuous agitation (300 rpm) and medium dripping (5 mL/min) at a ratio of 3 times the volume of the Formation. After 30 minutes of rest without agitation, the process continues with the step of Centrifugation at 1500 rpm for 15 mins., at 4°C. During this process the cochlear structures sediment, with complete discarding of the SN (supernatant) and slow resuspension of the pellet in Tris 10 mM, DOC 0.25%, CaCl2 2.5 mM at the same volume of the Formation. 5-Final Formulation. The SN (supernatant) is completely discarded and the sediment ("pellet", cochlear structures) is resuspended in Tris-CaCl2 at a volume equal to the Formation. All of these processes are performed in a closed-loop cycle under completely sterile conditions (Fig. 1).

### Example 5. Characterization of AFCo3-LPSd

The following were performed on tree variants of AFCo3 LPSd (2, 4, and 6 hours) and on AFCo3 LPSsd (not detoxified):
Optic microscope. To confirm the presence of tubular structures (Fig. 2).
Quantification of LPS. The content of LPS was evaluated with the determination of KDO through the TBA method (Tab. 2).

**Tab. 2. Quantification of LPS in AFCo3 and percentage of incorporation in the structure. Through the determination of 2-keto-3-deoxyoctonoic acid (KDO) using the thiobarbituric acid (TBA) method described by Osborn, the content of LPS was evaluated in each stage of obtaining the AFCo3 (A): in the initial resuspension (R), after the formation (F), and in the final formulation (I).**

| Formulation | LPS Concentration (mg/mL) | | |
|---|---|---|---|
| | **R** | **F** | **I** |
| **AFCo3 LPSd 2h** | 1 | 0.525 | 0.18 |
| **AFCo3 LPSd 4h** | 1 | 1 | 0.833 |
| **AFCo3 LPSd 6h** | 1 | 0.966 | 0.820 |
| **AFCo3 LPSsd** | 0.04 | 0.039 | 0.037 |
| **AFCo1** | 0.04 | 0.035 | 0.035 |

### Percentage of incorporation of LPS in the structure

The percentage was determined through the calculation (I x 100)/T wherein I is the concentration of LPS in the precipitate and T is the total concentration of LPS (Fig. 3).
Electrophoretic evaluation. Electrophoresis was performed in sodium dodecyl sulfate polyacrylamide gel (SDS-PAGE), using a gel concentrator at 5% (m/v) and gel separator at 15% (m/v). Silver-stain was utilized according to Tsai CM et al (Fig. 4).

### Results

Tubular structures were formed in all of the obtained formulations, of which the AFCo3 LPSd of 4 hrs. had the best structural appearance and also had the best inclusion of LPS in its structure (87%). In SDS-PAGE, homogeneity was observed in the electrophoretic pattern in all of the formulations, with a band up to the inferior limit of electrophoresis with equal molecular weight.

### Conclusion

The 4 hr. variant of AFCo3LPSd had the best appearance and best percentage of incorporation of LPS.

### Example 6. Inmunogenicity of AFCo3 LPSd variants

**Protocol for Immunization. In order to evaluate the immunogenicity of the** three variants of AFCo3 LPSd (2, 4, and 6 hours), BALB/c mice were immunized with two intramuscular (i.m.) doses of 50 µL each of OVA (10 µg) applied at 0 and 14 days and coadministered with 25 µg of AFCo3LPSd (variants 2, 4, and 6 hrs.) and 0.5 µg of AFCo3LPSsd (not detoxified). 0.5 µg of LPS in AFCo1 and DOC-Ca (sodium deoxycholate 0.25% / calcium 2.5 mM) were used as a positive control.
Method for obtaining and processing samples: The blood extraction was performed at 21 days after the last dose. In order to obtain the serum, the blood was extracted by penetrating the retro-orbital plexus, utilizing heparinized capillaries. The blood samples obtained were incubated for 1 hr. at 37°C, followed by centrifugation at 12500 g for 20 mins. in order to extract the serum. The samples were conserved at 20°C until their use.
Anti-OVA IgG detection through ELISA

### Reagents and Solutions:

- OVA, grade V purity (SIGMA St. Louis, MO, U.S.A.);
- Coating buffer solution: Na2CO3 11 mM, NaHCO3 35 mM (pH 9.6)
- Phosphate-buffered saline solution 0.15 M (pH 7.2);
- Blocking solution: Abovementioned phosphate-buffered saline solution, 1% Type B gelatin from bovine skin (SIGMA, St. Louis, MO, U.S.A.);
- Wash solution (WS): Abovementioned phosphate-buffered saline solution, 0.05% Tween-20 (v/v);
- Sample dilution solution: WS, 1% Type B gelatin from bovine skin (SIGMA, St. Louis, MO, U.S.A.);
- Peroxidase-conjugated anti-mouse IgG (SIGMA, St. Louis, MO, U.S.A.);
- Substrate buffer solution: Na2HPO4 52 mM, citric acid 25 mM (pH 5.6) and
- Stopping solution: H2SO4 2 M.

### Method:

- In 96-well plates with high bonding capability (Maxisorp, Nunc, U.S.A.), add 100 µL/well of OVA at a concentration of 10 µg/mL in above-described coating buffer solution and incubate all night at 4°C in a humid room;
- Wash three times with above-described phosphate-buffered saline solution;
- Add 200 µL/well of above-described blocking solution and incubate for 1 hour at room temperature in a humid room.
- Wash three times with above-described wash solution (WS);
- In above-described sample dilution solution, 1:100 dilution of the serum is performed and it is applied at 100 µL/well in duplicate for each one of the diluted samples, followed by incubation for 2 hours at 37°C;
- Wash three times with above-described wash solution (WS);
- The peroxidase-conjugated anti-mouse IgG diluted 1:2000 in above-described sample dilution solution is added in the amount of 100 µL/well;
- It is incubated for 1 hour at 37°C in a humid room;
- Wash five times with above-described wash solution (WS);
- Add 100 µL/well of hydrogen peroxide solution 0.01% (v/v) and 6 mg/mL of ortho-phenylenediamine (OPD) in above-described substrate buffer solution;
- It is incubated in darkness for 30 minutes.

The reaction is stopped by the addition of 50 µL of a stopping solution and the absorbance measurement (optical density) is performed at a wavelength of 492 nm in a plate reader (Titertek, Multiskan Plus).

### Expression and Calculation of the Results

The anti-OVA IgG results were expressed as optical density. In this assay, the samples were considered positive when above the value of the averages plus 2 standard deviations of the control samples (non-immunized groups).

### Results

The AFCo3LPSd formulation with a detoxification time of 4 hours, administered intramuscularly (i.m.) induced an efficient systemic immune response of anti-OVA IgG significantly superior to the response induced by formulations with 2 and 6 hours of detoxification and similar to that induced by AFCo3LPSsd (without detoxification) and to that by AFCo1 (Fig. 5). **Conclusion**. The 4 hour-variant of AFCo3LPSd was the variant that induced the best immunogenicity.

### Example 7. The intranasal administration of AFCo3LPSd 4 h induces anti-OVA and anti-TT IgG response in serum of immunized mice

**Protocol of Immunization**. C57BL/6 mice were immunized with three intranasal doses (i.n., 12.5 µL, via the nasal cavity) of OVA (20 µg) or tetanus toxoid (TT, 5 Lf (Limit of Flocculation)) at 0, 7, and 14 days, adjuvated with 25 µg of AFCo3LPSd 4h; 2 µg of AFCo3LPSsd; 2 µg of LPS in AFCo1, and the control was not immunized.
Method of serum extraction and processing: See example 6
Detection of anti-OVA IgG by ELISA: See example 6
Detection of anti-TT IgG by ELISA:

### Reagents and Solutions:

All of the reagents used are similar to those of Example 8, except that TT (lot-9007) produced under GMP conditions at the Finlay Institute was used for the coating.

### Method:

The method used was similar to that described in Example 6, except that TT at a concentration of 2 Lf/mL was used for the coating step.

### Expression and Calculation of the Results:

The anti-OVA and anti-TT IgG results were expressed as optical density. In this assay, the samples were considered positive when above the value of the averages plus 2 standard deviations of the control samples (non-immunized groups).

### Results

The intranasally administered AFCo3LPSd 4 h induces an efficient systemic immune response of anti-OVA and anti-TT IgG, similar to that induced in C57BL/6 mice immunized with AFCo1 and AFCo3LPSsd (not detoxified) (Fig. 6).

### Conclusion

Adjuvation with AFCo3 using LPSd via the mucosal route shows effectiveness with similar specific systemic response to that achieved with AFCo1 containing multiple MAMPs.

### Example 8. The intranasal administration of AFCo3LPSd 4 h induces anti-OVA and anti-TT IgG1 e IgG2c subclasses in the serum of immunized mice.

**Immunization Protocol**. C57BL/6 mice were immunized with three doses (0, 7, and 14) of OVA (20 µg) or TT (5 Lf) via i.n. (12.5 µL through each nasal cavity), administered with 25 µg of AFCo3LPSd 4 h, 2 µg AFCo3LPSsd (not detoxified) and 2 µg of LPS in AFCo1. The control group consisted of animals that were not immunized.
Method of serum extraction and processing: See example 6
Detection of anti-OVA and anti-TT IgG1 e IgG2c subclasses by ELISA:

### Reagents and Solutions:

OVA, grade V purity, SIGMA
TT (Lot-9007) produced under GMP conditions at Finlay Institute Peroxidase-conjugated biotinylated anti-mouse IgG1 (Amersham, LIFE SCIENCE)
Peroxidase-conjugated anti-mouse IgG2 (SouthernBiotech) Streptavidin-peroxidase (SIGMA, St. Louis, MO, U.S.A.)
The rest of the solutions: coating buffer solution, phosphate-buffered saline solution, blocking solution, wash solution (WS), sample dilution solution, substrate buffer solution, and stopping solution were the same as those described in Example 6.

### Method:

- In 96-well plates with high bonding capability (Maxisorp, Nunc, U.S.A.), coat with OVA (10 µg/mL) or TT (2 Lf/mL) in coating buffer solution and incubate all night at 4°C in a humid room.
- Wash three times with above-described phosphate-buffered saline solution.
- Add 200 µL/well of above-described blocking solution and incubate for 1 hour at room temperature in a humid room.
- Wash three times with above-described wash solution (WS);
- In above-described sample dilution solution, 1:100 dilution of the serum is performed and it is applied at 100 µL/well in duplicate for each one of the diluted samples, followed by incubation for 2 hours at 37°C.
- Wash three times with above-described wash solution (WS).
- Add 100 µL/well of conjugated IgG1 1:5000 and IgG2c 1:4000. Incubate for 1 hour in a humid room.
- For the biotinylated IgG1, this is followed by washing the plates three times and adding 100 µL/well of the second conjugate (streptavidin-peroxidase) 1:2500 in the sample dilution solution and incubating for 30 minutes at 37°C.
- Wash five times with above-described wash solution (WS).
- Add 100 µL/well of hydrogen peroxide solution 0.01% (v/v) and 6 mg/mL of ortho-phenylenediamine (OPD) in above-described substrate buffer solution. It is incubated in darkness for 30 minutes.
- The reaction is stopped by the addition of 50 µL of a stopping solution.
- The absorbance measurement (optical density) is performed at 492 nm in a plate reader (Titertek, Multiskan Plus).

### Expression and Calculation of the Results:

The anti-OVA and anti-TT IgG1 and IgG2c results were expressed as optical density. In this assay, the samples were considered positive when above the value of the averages plus 2 standard deviations of the control samples (non-immunized groups).

### Results

The intranasally administered AFCo3LPSd 4 h induces an efficient response of specific anti-OVA and anti-TT IgG1 and IgG2c, similar to that induced in C57BL/6 mice immunized with AFCo1 and AFCo3LPSsd (without detoxification) (Fig. 7).

### Conclusion

Adjuvation with AFCo3 using LPSd via the mucosal route demonstrates effectiveness, with a similar pattern of subclasses of specific IgG as AFCo1 which contains multiple MAMPs.

### Example 9. The intramuscular administration of AFCo3LPSd 4 h induces a specific response of anti-BSA IgG in the serum of immunized mice.

**Immunization Protocol**. C57BL/6 mice were immunized with two i.m. doses of bovine serum albumin (BSA, 20 µg) at 0 and 14 days, adjuvated with 25 □g of AFCo3LPSd 4 h, 0.5 □g of AFCo3LPSsd (not detoxified), 0.5 □g of LPS in AFCo1, and a control group of animals was not immunized.
**Method of serum extraction and processing**. See example 6
Detection of anti-BSA IgG by ELISA:

### Reagents and Solutions.

All of the reagents used were similar to those described in Example 6, except that BSA Fraction V (SIGMA, St. Louis, MO, U.S.A.) was used.

### Method.

- The method used was similar to that described in Example 6, except that BSA was used at a concentration of 10 µg/mL in the coating step.
- Expression and Calculation of the Results.
- The anti-BSA IgG results were expressed as optical density. In this assay, the samples were considered positive when above the value of the averages plus 2 standard deviations of the control samples (non-immunized groups).

### Results

The intramuscularly administered AFCo3-LPSd 4 h induces a specific, efficient response of anti-BSA IgG, similar to that induced when the adjuvation is performed with AFCo1 or AFCo3LPSsd (without detoxification) (Fig. 8).

### Conclusion

Adjuvation with AFCo3 (AFCo3LPSd 4 h) using LPSd via the parenteral route demonstrates effectiveness, with a similar specific systemic response as that achieved with AFCo1 which contains multiple MAMPs.

### Example 10. The intramuscular administration of AFCo3LPSd 4 h induces a specific response of anti-BSA IgG in the serum of immunized mice.

**Immunization Protocol**. C57BL/6 mice were immunized with two i.m. doses of BSA (20 µg) at 0 and 14 days, adjuvated with 25 □g of AFCo3LPSd 4 h, 0.5 □g of AFCo3LPSsd (not detoxified), 0.5 □g of LPS in AFCo1, and a control group of animals was not immunized.
Method of serum extraction and processing: See example 6
Detection of anti-BSA IgG1 e IgG2c subclasses by ELISA:

### Reagents and Solutions.

Similar to Example 8 except for the use of BSA Fraction V (SIGMA, St. Louis, MO, U.S.A.)

### Methods.

Similar to Example 8, except for the coating which was performed using BSA (10 µg/mL).

### Expression and Calculation of the Results.

The results of the anti-BSA IgG1 and IgG2c subclasses were expressed as optical density. In this assay, the samples were considered positive when above the value of the averages plus 2 standard deviations of the control samples (non-immunized groups).

### Results.

The intramuscularly administered AFCo3-LPSd 4 h induces a specific, efficient systemic immune response of anti-BSA IgG1 and IgG2c subclasses in immunized C57BL/6 mice, with a similar pattern of subclasses as AFCo1 and non-detoxified LPS (Fig. 9)

### Conclusion

Adjuvation with AFCo3 using LPSd via the mucosal route demonstrates effectiveness, with a similar pattern of specific IgG subclasses as AFCo1 which contains multiple MAMPs.

### Example 11. Obtaining AFCo3-G1

2-bromo-5-(2-bromo-2-nitrovinyl)-furan is a bioactive compound belonging to the nitrovinylfuran family, obtained from furfural with a large variety of pharmacological actions (wide-spectrum antifungal and antibacterial). Its incorporation into AFCo3 utilized the 4 hour variant of detoxified LPS (LPSd) and G1 adjusted to a concentration of 2 mg/mL in the Resuspension Solution (Tris 30mM, 1% of DOC (^{··}Resuspension^{··} process). The remainder of the steps were performed as described in example 3. In each step, a sample was obtained to determine the G1 and, thereby, to confirm the inclusion in AFCo3.
**Determination of G1**: The determination of G1 was carried out by ultraviolet-visible spectrophotometry.

### Reagents and Solutions:

G1: Centro de Bioactivos Quimicos (CBQ) (Chemical Bioactive Center), Villa Clara, Cuba
Dimethylformamide: Sigma- Aldrich, Germany
Tris-Ca: Tris, 10 mM and CaCl 2.5 mM

### Methods:

- Prepare a stock solution of G1 at 1 mg/mL in Dimethylformamide
- In U-bottom 96-well plates (Costar, USA), using a stock solution of G1, prepare a G1 curve at a concentration from 0.02 to 0.2 mg/mL in Tris-Calcium, adding 200 µL per well.
- In other wells, add 200 µL of the samples for analysis (supernatants obtained in each step of incorporation of G1 in the AFCo3)
- The absorbance measurement (optical density) is performed at a wavelength of 340 nM in a plate reader (Titertek, Multiskan Plus).

### Results: Expression and Calculation

The concentration of G1 in the samples was evaluated through the performance trend parallel to the curve calculating the linear fit equation, as well as the R2 coefficient. The G1 curve was defined as the independent variable (X) and the analyzed samples were defined as the dependent variable (y). The final concentration was determined by substituting in the formula obtained with the values of the curve (Tab. 3).

Percentage of inclusion of LPS G1 in the structure: This was determined through the calculation (I x 100)/T wherein I is the concentration of G1 in the precipitate and T is the concentration of G1 in total (Tab. 3).

**Tab. 3. Quantification of G1 in AFCo3 and percentage of incorporation in the structure. The content of G1 in the supernatant of the wash, in the formed AFCo3 (T), and in the precipitate (1) upon centrifuging. The concentration of G1 in the samples was evaluated by ultraviolet-visible spectrophotometry, through the performance trend parallel to the G1 curve, calculating the linear fit equation as well as the R2 coefficient. The G1 curve was defined as the independent variable (X) and the analyzed samples were defined as the dependent variable (y). The final concentration was determined by substituting in the formula obtained with the values of the curve. The percentage of incorporation of LPS in AFCo3 was determined through the calculation ((I x 100)/T) wherein I is the concentration of G1 in the precipitate and T is the concentration of G1 in total.**

| G1 Curve in Tris-Ca Conc (mg/mL) | Optical Density 1 | Optical Density 2 | Optical Density prom | Optical Density prom |
|---|---|---|---|---|
| White (Tris-Cal) | 0.311 | 0.32 | 0.316 | white |
| 0.02 | 0.424 | 0.412 | 0.418 | 0.10 |
| 0.04 | 0.527 | 0.557 | 0.542 | 0.23 |
| 0.06 | 0.608 | 0.595 | 0.602 | 0.29 |
| 0.1 | 0.688 | 0.692 | 0.690 | 0.37 |
| 0.14 | 0.843 | 0.853 | 0.848 | 0.53 |
| 0.16 | 0.931 | 0.944 | 0.938 | 0.62 |
| 02 | 1 | 0.989 | 0.995 | 0.68 |

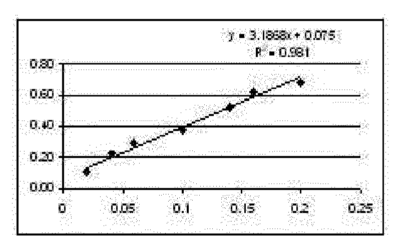

| Formulations | Optical Density 1 | Optical Density 2 | Optical Density prom | Optical Density prom white | Conc (mg/mL) | %Incorporation |
|---|---|---|---|---|---|---|
| SN wash | 0.947 | 0.924 | 0.936 | 0.620 | 0.171 | |
| AFCo3 formed | 0.919 | 0.869 | 0.894 | 0.426 | 1.101 | |
| SN total | 0.662 | 0.762 | 0.712 | 0.396 | 0.101 | |
| Pellet | 0.872 | 0.872 | 0.872 | 0.404 | 1.032 | 100 |

### Conclusion:

AFCo3 G1 was obtained with a percentage rate of 100% incorporation within the structure.

### Example 12. Treatment with AFCo3-G1 in homing pigeons infected with the avian pox virus

### Animal model

12 homing pigeons (Columbia livia), 28-56 days of age and infected naturally with the avian pox virus with clinically typical lesions in the nasal, peribuccal, and periocular regions, as well as in the inferior extremities. The number of lesions varied between animals, with a median of 6 lesions per bird, a minimum value of 4, and a maximum value of 13.

### Treatment

When the treatment was initiated, the rash had been developing for approximately 21 days, and at that time 76% (13/17) of the pigeons were affected and one pigeon had died from it after a progressive debilitation.
To all of the surviving pigeons (16 in total, 12 with lesions and 4 that were clinically healthy), 100 µL of AFCo3-G1 was applied subcutaneously and, simultaneously, 30 µL of the same product was applied nasally (15 µL via the nasal cavity). After seven days, 30 µL was applied only via the nasal route. At 7, 14, 21, 28 and 35 days, the presence of lesions in the treated animals was evaluated and recorded.

### Results

None of the clinically healthy pigeons that were treated with AFCo3-G1 developed typical lesions of the avian pox nor any observed adverse reactions. In the diseased pigeons that received the treatment, there was an observed gradual reduction in number and severity of the lesions at the evaluation seven days after the treatment (first evaluation). At 21 days, only two pigeons still had an active lesion and at 35 days, these had completely healed.

### Conclusions

AFCo3-G1 was effective in treating avian pox in homing pigeons when administered subcutaneously and nasally.

## Claims

1. Vaccine adjuvant **characterized by** cochlear structures that are self-assembled, coiled, and that have multiple layers obtained from structures that are neither proteolipidic nor lipidic, and said cochlear structures contain molecular patterns associated with microbes, deoxycholate, and divalent cations in an adequate vehicle.

2. Vaccine adjuvant according to claim 1, wherein the microbe-associated molecular patterns can be purified of microbes or obtained in a recombinant or synthetic form in which the toxins are detoxified and only one or various, but with synergistic action, are included.

3. Vaccine adjuvant according to claim 1, wherein its immunopotentiatory capabilities are utilized in medicine for humans and animals, preferably mammals, fish, and birds, and for which the human diseases can be chronic diseases that are either infectious or noninfectious.

4. Vaccine adjuvant according to claim 3 with its immunopotentiatory capabilities administered mucosally or parenterally or by both routes simultaneously, with preferable mucosal administration via the oral or nasal route and the parenteral administration via the intramuscular, subcutaneous, or topical route.

5. Vaccine adjuvant according to claim 1, wherein antibiotics/chemotherapeutic agents are incorporated in the cochlear structures for their application in animals, including humans.

6. Vaccine adjuvant according to claim 5, wherein the antibiotic/chemotherapeutic agent is 2-bromo-5-(2-bromo-2-nitrovinyl)-furan (G1), preferably for its use in mammals, fish, and birds, and is administered mucosally or parenterally or via both routes simultaneously, and wherein the mucosal administration is via the oral or nasal route and the parenteral administration is via the intramuscular, subcutaneous, or topical route.

7. Vaccine adjuvant according to claims 5-6, wherein the cochlear structures containing antibiotics/chemotherapeutic agents are utilized in chronic human diseases that are either infectious or noninfectious.

8. Vaccine composition **characterized by** self-assembled, coiled, and multilayered cochlear structures obtained from structures that are neither proteolipidic nor lipidic and antigens, with said structures containing molecular patterns associated with microbes, particular antigens, deoxycholate, and divalent cations in an adequate vehicle.

9. Vaccine composiiton according to claim 8, wherein the microbe-associated molecular patterns can be purified of microbes, obtained in a recombinant or synthetic form, wherein the toxins are detoxified and only one or various, but with synergistic action, are included.

10. Vaccine composition according to claims 8-9, wherein the particular antigens come from infectious agents that affect animals including humans, preferably mammals, fish, and birds, and for which the human diseases can be chronic diseases that are either infectious or noninfectious.

11. Vaccine composition according to claims 8-10, wherein said compositions are applied mucosally, parenterally, or via both routes simultaneously, preferably wherein the mucosal administration is via the oral, nasal, or sublingual route and the parenteral administration is via the intramuscular, subcutaneous, or transdermal route.

12. Vaccine composition according to claim 8, wherein the antibiotics/chemotherapeutic agents are incorporated into the cochlear structures for their immunotherapeutic application in animals including humans, preferably 2-bromo-5-(2-bromo-2-nitrovinyl)-furan (G1), for its application in mammals, fish, and birds, administered mucosally or parenterally or via both routes simultaneously, wherein the mucosal administration is via the oral or nasal route and the parenteral administration is via the intramuscular, subcutaneous, or transdermal route, preferably for the treatment of chronic diseases that are either infectious or noninfectious.

13. Vaccine composition according to claims 8 to 12, wherein the antigens are provided by the death of the pathogens caused by the combined effect of the antibiotics/immunotherapeutic agents and immunopotentiation.

14. Sterile method for obtaining vaccine adjuvants containing coiled cochlear structures, self-assembled of multiple layers obtained from structures that are neither proteolipidic nor lipidic or of those with the optional presence of antibiotics.

15. Sterile method for obtaining vaccine compositions containing coiled cochlear structures, self-assembled of multiple layers obtained from structures that are neither proteolipidic nor lipidic or of those and antigens with the optional presence of antibiotics.
